(19) Europäisches Patentamt

European Patent Office

Office européen des brevets

(11) EP 1 264 182 B1

(12) **EUROPÄISCHE PATENTSCHRIFT**

(45) Veröffentlichungstag und Bekanntmachung des Hinweises auf die Patenterteilung:
**16.11.2005 Patentblatt 2005/46**

(21) Anmeldenummer: **01929228.3**

(22) Anmeldetag: **05.03.2001**

(51) Int Cl.⁷: $G01N\ 33/566$, G01N 33/569, A61K 38/00, A61K 31/00, A61K 39/00

(86) Internationale Anmeldenummer:
**PCT/DE2001/000817**

(87) Internationale Veröffentlichungsnummer:
**WO 2001/065257 (07.09.2001 Gazette 2001/36)**

(54) **VERFAHREN ZUR ANALYSE VON EXOGENER UND ENDOGENER ZELL-AKTIVIERUNG**

METHOD FOR THE ANALYSIS OF EXOGENIC AND ENDOGENIC CELL ACTIVATION

PROCEDE D'ANALYSE D'ACTIVATION CELLULAIRE EXOGENE ET ENDOGENE

(84) Benannte Vertragsstaaten:
**AT BE CH CY DE DK ES FI FR GB GR IE IT LI LU MC NL PT SE TR**

(30) Priorität: **05.06.2000 DE 10027308**

(43) Veröffentlichungstag der Anmeldung:
**11.12.2002 Patentblatt 2002/50**

(73) Patentinhaber: **Schmitz, Gerd**
**93042 Regensburg (DE)**

(72) Erfinder:
• **SCHMITZ, Gerd Universität Regensburg**
**93042 Regensburg (DE)**
• **GÖTZ, Alexandra**
**93053 Regensburg (DE)**

(74) Vertreter: **Schüssler, Andrea**
**Kanzlei Huber & Schüssler**
**Truderinger Strasse 246**
**81825 München (DE)**

(56) Entgegenhaltungen:
**WO-A-95/14039**

• **GOETZ A ET AL: "Ligand specific heteromeric CD14-clustering in inflammation." JOURNAL OF ENDOTOXIN RESEARCH, Bd. 6, Nr. 2, 2000, Seite 106 XP001024379 6th Conference of the International Endotoxin Society;Paris, France; August 24-27, 2000 ISSN: 0968-0519**
• **SCHMITZ G ET AL: "Ceramide and LPS induce heteromeric CD14-clustering with raft associated receptors on monocytes: An early indicator in inflammation and atherosclerosis?" IMMUNOLOGY LETTERS, Bd. 73, Nr. 2-3, September 2000 (2000-09), Seiten 116-117, XP001024378 24th European Immunology Meeting of the European Federation of Immunological Societies;Poznan, Poland; September 23-26, 2000 ISSN: 0165-2478**
• **VIRIYAKOSOL SUGANYA ET AL: "Structure-function analysis of CD14 as a soluble receptor for lipopolysaccharide." JOURNAL OF BIOLOGICAL CHEMISTRY, Bd. 275, Nr. 5, 4. Februar 2000 (2000-02-04), Seiten 3144-3149, XP001024952 ISSN: 0021-9258**
• **SCHWANDNER RALF ET AL: "Peptidoglycan- and lipoteichoic acid-induced cell activation is mediated by toll-like receptor 2." JOURNAL OF BIOLOGICAL CHEMISTRY, Bd. 274, Nr. 25, 18. Juni 1999 (1999-06-18), Seiten 17406-17409, XP001024953 ISSN: 0021-9258**

**Beschreibung**

Die vorliegende Erfindung betrifft ein Verfahren zur Analyse von exogener und endogener Zell-Aktivierung, umfassend die Messung der Assemblierung von Rezeptoren in einem CD14 umfassenden Rezeptor-Cluster. Dies erfolgt durch die Messung des Energietransfers zwischen den einzelnen Rezeptoren, wobei eine Messung mittels Fluoreszenz Resonanz Energietransfer (FRET) bevorzugt ist. Die vorliegende Erfindung betrifft femer ein Verfahren zur Diagnose systemischer Inflammationen, wie Sepsis, Arteriitis oder Autoimmunerkrankungen, oder einer arteriosklerotischen oder entzündlichen Erkrankung der Koronararterien (CAD, wie Angina Pectoris, Herzinfarkt oder Coronarsclerosen) bzw. der Cerebralarterien (wie Schlaganfall) oder einer Vorstufe einer dieser Erkrankungen, das auf der Messung der Assemblierung von Rezeptoren in einem CD14 umfassenden Rezeptor-Cluster basiert, sowie die Verwendung von Verbindungen, die das Clustern von CD14 verhindern, zur Behandlung dieser Erkrankungen.

CD14, ein wichtiger Oberflächenrezeptor auf Monozyten, spielt eine bedeutende Rolle bei Entzündungsreaktionen als Antwort auf bakterielle Pathogene. Neuere Daten deuten daraufhin, daß die Expression von CD14 bei einer systemischen Entzündung verändert ist. Ferner wird die Expression von CD14 bei einer HMG-CoA-Reduktase-Inhibitor-Therapie erniedrigt und die Expansion einer stärker differenzierten Monozyten-Subpopulation mit hoher Expression von CD14 und des Fcy-Rezeptors IIIa (Fcγ-RIIIa, CD16a) korreliert mit einem atherogenen Lipoproteinprofil, was auf eine regulatorische Funktion dieses Rezeptors sowohl bei einer systemischen Entzündung als auch Erkrankungen der Koronararterien hinweist.

Lipopolysaccharid (LPS) ist zwar der Hauptligand von CD14, neuere Daten zeigen jedoch, daß noch weitere Stoffe an CD14 binden, unter anderem Lipoteichonsäure, anionische Phospholipide, lösliches Peptidoglycan, Muramyldipetid, Polymannuronsäure, Lipoarabinomannan und weitere Produkte mikrobiellen Ursprungs. Kürzlich wurde gezeigt, daß die Streß-induzierbaren Mitglieder der Heat Shock Protein 70 (HSP70) Familie an Monozyten die Zytokin-Produktion durch einen CD14-vermittelten Signaltransduktionsweg induzieren. Außerdem ist bekannt, daß HSP70 ein Ceramid-Derivat, Sulfogalactosylceramid (SG-cer) bindet.

Da es sich bei CD14 um einen Glycosylphosphatidylinositol-verankerten Rezeptor (GPI-R) ohne Transmembran-Domäne handelt, wurde davon ausgegangen, daß weitere Membranproteine an der Signaltransduktion beteiligt sind, da CD14 alleine kein Signal weiterleiten kann. Es spricht vieles dafür, daß eines (oder mehrere) Mitglieder der "Toll like"-Rezeptor (TLR) -Familie, insbesondere TLR4 und TLR2, an der LPS-induzierten Signaltransduktion beteiligt sind. Genetische und Spezies-spezifische Unterschiede bei der Erkennung von Lipid A deuten daraufhin, daß TLR4 das wesentliche für LPS Signalgebende Molekül ist. Eine direkte physikalische Interaktion von LPS oder CD14 mit TLRs konnte bisher allerdings nicht nachgewiesen werden. Verschiedene andere GPI-Rezeptoren, wie z.B. der Urokinase-Plasminogen-Aktivator-Rezeptor (uPA-R, CD87) und Decay Accelerating Factor (DAF, CD55) und Protectin (CD59) assoziieren ebenfalls - ähnlich wie CD14 - mit Detergenz-unlöslichen, mit Glycolipid angereicherten Domänen (DIGs). Es zeigte sich, daß es sich bei diesen DIGs um Cholesterin- und Sphingolipidreiche Membran-Domänen handelt, die auch als "Rafts" bezeichnet werden. Kleine "Raft"-Domänen führen nach der Rezeptor-Aggregation zur Bildung größerer Rezeptor-Cluster bzw. größerer Raft-Domänen, die mit Signal-gebenden Verbindungen auf der Zytosol-Seite assoziieren.

Die zentrale Rolle von CD14 in der Aktivierung von Monozyten deutet darauf hin, daß es weitere Liganden von CD14 gibt. Außerdem ist kaum etwas über das Potential der verschiedenen CD14-Liganden hinsichtlich der Induktion von spezifischen Rezeptorkomplexen bzw. -Clustem, beispielsweise auf der Oberfläche von menschlichen Monozyten, bekannt, weil bisher keine zuverlässigen und einfachen Verfahren zur Verfügung stehen, die die Messung einer solchen Bildung von Rezeptor- Clustern bzw. - indirekt - der dadurch ausgelösten Zell-Aktivierung erlauben.

Somit liegt der vorliegenden Erfindung das technische Problem zugrunde, Mittel bereitzustellen, die die Analyse von exogener oder endogender Zell-Aktivierung, z. B. einer zur Freisetzung von Entzündungen fördemden Mediatoren oder zu Atherosklerose führenden Zellaktivierung, erlauben.

Die Lösung dieses technischen Problems wird durch die Bereitstellung der in den Patentansprüchen gekennzeichneten Ausführungsformen erzielt. Es wurde gefunden, daß durch die Messung der Assemblierung von Rezeptoren in einem CD14 umfassenden Rezeptor-Cluster die Zellaktivierung bestimmt werden kann. Dies wurde mittels mehrerer Meßverfahren gezeigt, wobei insbesondere auf die Messung des Energietransfers zwischen den Rezeptoren, vorzugsweise durch Fluoreszenz Resonanz Energietransfer (FRET) hingewiesen wird. Hierbei wurde das Clustern des Glycosylphosphatidylinosit-verankerten Endotoxinrezeptors CD14 gemessen, der - wie bereits vorstehend diskutiert - eine wichtige Rolle bei einer Entzündungsreaktion in Monozyten als Reaktion auf Lipopolysaccharid (LPS) spielt. Es wurde außerdem gefunden, daß Ceramid - ein Bestandteil von atherogenen Lipoproteinen - als extrazellulärer Ligand von CD14 wirkt. Anhand des Vergleichs der Wirkungen von LPS und Ceramid wurden auf dem Clustern von CD14 mit Co-Rezeptoren basierende Rezeptor/Liganden-Interaktionen untersucht. Es zeigte sich, daß sowohl LPS als auch Ceramid die Co-Assemblierung von CD14, Komplementrezeptor 3 (CD11 b/CD18), CD36 und DAF (CD55) induzierten. Interessanterweise induzierte nur LPS die Co-Assemblierung mit dem "Toll like" Rezeptor 4 (TLR 4), Fcyllla (CD16a) und dem Integrin-assoziierten Protein (IAP) CD81, während Ceramid das Clustern mit CD47 (einem weiteren IAP) induzierte. Dies deutet auf die Existenz zweier verschiedener zellulärer Wege hin, die mit der Assemblierung von kongenitalen Rezeptcrkompiexen in "Rafts" in Zusammenhang siehen. Die Bindung von LPS an CD14 ist bei Sepsis von einer Freisetzung von entzündungsfördernden Mediatoren begleitet und die Bindung von Ceramid an CD14 steht mit Atherosklerose in Zusammenhang. Ceramid scheint ein Modulator der Immunfunktion bei Atherosklerose zu sein.

Gegenstand der vorliegenden Erfindung ist somit ein Verfahren zur Analyse von exogener und endogener Zell-Aktivierung, umfassend die Messung der Assemblierung von Rezeptoren in einem CD14 umfassenden Rezeptor-Cluster.

Der verwendete Ausdruck "exogene Zell-Aktivierung" bezeichnet die Induktion membranassoziierter Signaltransduktionskaskaden durch exogene (körperfremde) Stimuli.

Der verwendete Ausdruck "endogene Zell-Aktivierung" bezeichnet die Induktion membranassoziierter Signaltransduktionskaskaden durch endogene (körpereigene) Stimuli.

Der verwendete Ausdruck "Messung der Assemblierung von Rezeptoren in einem CD14 umfassenden Rezeptor-Cluster" umfaßt Messung eines Energietransfers zwischen den Rezeptoren, vorzugsweise eine Messung mittels FRET und ganz besonders bevorzugt eine Messung, in der FRET mit Durchflußzytometrie (Flow Cytometric Energy Transfer, FCET) kombiniert ist. Die Messung eines Energietransfers ist dabei so zu verstehen, daß nicht der Energietransfer der assemblierenden Rezeptoren direkt gemessen wird, sondem der zwischen Antikörpern, die an die verschiedenen Rezeptoren binden, wobei der Energietransfer ein Anzeichen für eine Co-Assemblierung der entsprechenden Rezeptoren, d.h. für eine Ortsnähe zueinander von etwa 2-10 nm, und somit für eine Zell-Aktivierung ist.

In einer bevorzugten Ausführungsform des erfindungsgemäßen Verfahrens erfolgt die Messung der Assemblierung von Rezeptoren in einem CD14 umfassenden Rezeptor-Cluster mittels der Messung eines Energietransfers zwischen den Rezeptoren, vorzugsweise mittels FRET, besonders bevorzugt mittels FRET in Kombination mit FCET. Ganz besonders bevorzugt ist ein erfindungsgemäßes Verfahren bei dem eine Energietransfer-Effizienz oder ein Energietransfer-Parameter ($Et_p$) > 5% ein Anzeichen von endogener oder exogene Zell-Aktivierung ist. Die Messung dieses Werts kann gemäß dem nachstehenden Beispiel 1 (1) erfolgen.

In einer weiteren bevorzugten Ausführungsform des erfindungsgemäßen Verfahrens umfaßt der CD14 umfassende Rezeptor-Cluster außerdem LPS-assoziierte Proteine, z.B. TLR-4, Integrin-assoziierte Proteine, z.B. CD81 und CD47, G-Protein-Rezeptoren, Komplement-regulierende Proteine, z.B. CD11b, CD18 und CD55, Fcy-Rezeptoren, z.B. CD16a, CD32 und CD64, und/oder Scavenger Rezeptoren, z.B. CD36. In einer mehr bevorzugten Ausführungsform des erfindungsgemäßen Verfahrens wird der Energietransfer zwischen einem Antikörper gegen ein vorstehendes Protein (Rezeptor), insbesondere CD11b, und einem anti-CD14-Antikörper gemessen.

Die vorliegende Erfindung betrifft ferner ein Verfahren zur Diagnose systemischer Inflammationen, wie Sepsis, Arteriitis oder Autoimmunerkrankungen, oder einer arteriosklerotischen oder entzündlichen Erkrankung der Koronararterien (CAD, wie Angina Pectoris, Herzinfarkt oder Coronarsclerosen) bzw. der Cerebralarterien (wie Schlaganfall) oder einer Vorstufe einer dieser Erkrankungen, das dadurch gekennzeichnet ist, daß eine Messung der Assemblierung von Rezeptoren auf Monozyten einer Patientenprobe (z.B. frisches Lithium-Heparin Vollblut, ca. 1 ml) gemäß dem erfindungsgemäßen, vorstehend beschriebenen Verfahren durchgeführt wird, wobei z. B. ein Energietransfer, vorzugsweise eine Energietransfer-Effizienz oder ein Energietransfer-Parameter ($Et_p$) > 5% ein Anzeichen für systemische Inflammationen, wie Sepsis, Arteriitis oder Autoimmunerkrankungen, oder für eine arteriosklerotische oder entzündliche Erkrankung der Koronararterien (CAD, wie Angina Pectoris, Herzinfarkt oder Coronarsclerosen) bzw. der Cerebralarterien (wie Schlaganfall) oder für eine Vorstufe einer dieser Erkrankungen ist.

Da durch das Clustern der vorstehend beschriebenen Rezeptoren die vorstehend diskutierten Erkrankungen, z.B. systemische Inflammationen, wie Sepsis, Arteriitis oder Autoimmunerkrankungen, oder eine arteriosklerotische oder entzündliche Erkrankung der Koronararterien (CAD, wie z.B. Angina Pectoris, Herzinfarkt oder Coronarsclerosen) bzw. der Cerebralarterien (wie z.B. Schlaganfall) ausgelöst werden können, kann durch die Verhinderung dieses Clustems eine Therapie oder Prävention dieser Erkrankungen erfolgen.

Somit betrifft die vorliegende Erfindung auch die Verwendung einer Verbindung, die exogene entzündungsfördernde Moleküle wie LPS, Lipoteichonsäure, Ceramide und assoziierte Proteine z.B. SG-cer/HSP70, endogene entzündungsvermitteinde Moleküle, und/oder Phospholipide, z.B. Phosphatidylinositol oder Posphotidylethanolamin, neutralisiert, zur Behandlung systemischer Inflammationen, wie Sepsis, Arteriitis oder Autoimmunerkrankungen, oder einer arteriosklerotischen oder entzündlichen Erkrankung der Koronararterien (CAD, wie Angina Pectoris, Herzinfarkt oder Coronarsclerosen) bzw. der Cerebralarterien (wie Schlaganfall) oder einer Vorstufe dieser Erkrankungen. Gleiches gilt auch für Verbindungen, die LPS-assoziierte Rezeptoren, z. B. CD14 und TLR-4, Komplement regulierende Proteine z. B. CD11b, CD18 und CD55, Fcy-R, z. B. CD16a, CD32 und CD64, Integrin-assoziierten Proteine, z. B. CD81 und CD47, und/oder Scavenger Rezeptoren, z. B. CD36, neutralisieren. Der verwendete Ausdruck "neutralisierende Verbindung" betrifft jede Verbindung, die an diese Liganden bzw. Rezeptoren so binden oder diese so verändern kann, daß das Clustern von CD14 mit den anderen Rezeptoren vollständig oder zumindest wesentlich blockiert wird. Die Identifizierung solcher Substanzen und die Untersuchung ihrer Wirksamkeit kann gemäß Routineverfahren erfolgen, z.B. durch Messung, ob diese einen Energietransfer verhindern. Vorzugsweise ist die Ceramid neutralisierende Verbindung eine C24:1 neutralisierende Verbindung. Die Wirkung kann beispielsweise darauf beruhen, daß die Bindung der CD14-Liganden an CD14 blockiert und somit das Clustern verhindert wird. Dies kann beispielsweise dadurch erreicht werden, daß Antikörper verwendet werden, die entweder an den Rezeptor-Liganden selbst oder den Rezeptor (CD14 und/oder einen der vorstehend diskutierten Rezeptoren) derart binden, daß die Bindung des Liganden an seinen Rezeptor blockiert wird. Andererseits kann dies auch durch Verabreichung eines kompetitiven Analogstoffes (beispielsweise eines Antagonisten oder des extrazellulären (löslichen) Teils des Rezeptors) erreicht werden, wobei durch die (bevorzugte) Bindung des Liganden an das Rezeptoranalogon bzw. die (bevorzugte) Bindung des Antagonisten an den natürlichen Rezeptor die Bindung des biologisch aktiven Liganden an den natürlichen Rezeptor verringert oder ganz aufgehoben wird.

In einer bevorzugten Ausführungsform ist die neutralisierende Verbindung ein spezifisch bindender Antikörper, vorzugsweise ein monoklonaler Antikörper, oder ein Fragment davon.

Verfahren zur Gewinnung solcher Antikörper sind dem Fachmann bekannt und umfassen beispielsweise bezüglich polyklonaler Antikörper die Verwendung der vorstehend beschriebenen Liganden oder Rezeptoren, z.B. CD14, oder eines Fragments davon als Immunogen zur Immunisierung geeigneter Tiere und die Gewinnung von Serum. Verfahren zur Herstellung monoklonaler Antikörper sind dem Fachmann ebenfalls bekannt. Dazu werden beispielsweise Zell-Hybride aus Antikörper produzierenden Zellen und Knochenmark-Tumorzellen hergestellt und kloniert. Anschließend wird ein Klon selektiert, der einen Antikörper produziert, der für das gewünschte Immunogen spezifisch ist. Dieser Antikörper wird dann hergestellt. Beispiele von Zellen, die Antikörper produzieren, sind Milzzellen, Lymphknotenzellen, B-Lymphozyten etc. Beispiele von Tieren, die zu diesem Zweck immunisiert werden können, sind Mäuse, Ratten, Pferde, Ziegen und Kaninchen. Die Myelomzellen lassen sich aus Mäusen, Ratten, Menschen oder anderen Quellen erhalten. Die Zellfusion kann man beispielsweise durch das allgemein bekannte Verfahren von Köhler und Milstein durchführen. Die durch Zellfusion erhaltenen Hybridome werden mittels des Immunogens nach dem Enzym-Antikörper-Verfahren oder nach einem ähnlichen Verfahren selektiert. Klone werden beispielsweise mit dem Grenz-Verdünnungsverfahren erhalten. Die erhaltenen Klone werden BALB/c-Mäusen intraperitoneal implantiert, nach 10 bis 14 Tagen wird der Ascites der Maus entnommen, und der monoklonale Antikörper durch bekannte Verfahren (beispielsweise Ammoniumsulfatfraktionierung, PEG-Fraktionierung, Ionenaustauschchromatographie, Gelchromatographie oder Affinitätschromatographie) gereinigt. Der gewonnene Antikörper kann direkt verwendet werden oder es kann ein Fragment davon verwendet werden. In diesem Zusammenhang bedeutet der Begriff "Fragment" alle Teile des monoklonalen Antikörpers (z.B. Fab-, Fv- oder "single chain Fv"-Fragmente), welche die gleiche Epitopspezifität wie der vollständige Antikörper aufweisen.

In einer besonders bevorzugten Ausführungsform ist der genannte monoklonale Antikörper ein aus einem Tier (z.B. Maus) stammender Antikörper, ein humanisierter Antikörper oder ein chimärer Antikörper oder ein Fragment davon. Chimären, menschlichen Antikörpern ähnelnde oder humanisierte Antikörper besitzen eine herabgesetzte potentielle Antigenität, jedoch ist ihre Affinität gegenüber dem Ziel nicht herabgesetzt. Die Herstellung von chimären und humanisierten Antikörpern bzw. von den menschlichen Antikörpern ähnelnden Antikörpern wurde in der Literatur ausführlich beschrieben. Humanisierte Immunglobuline weisen variable Grundgerüstbereiche auf, die im wesentlichen von einem humanen Immunglobulin stammen (mit der Bezeichnung Akzeptor-Immunglobulin) und die Komplementarität der determinierenden Bereiche stammt im wesentlichen von einem nicht-menschlichen Immunglobulin (z.B. von der Maus) (mit der Bezeichnung Donor-Immunglobulin). Die (der) konstante(n) Bereich(e) stammt/stammen, falls vorhanden, auch im wesentlichen von einem menschlichen Immunglobulin. Bei der Verabreichung an menschliche Patienten bieten die erfindungsgemäßen humanisierten (sowie die menschlichen) Antikörper eine Reihe von Vorteilen gegenüber Antikörpern von Mäusen oder anderen Spezies: (a) das menschliche Immunsystem sollte das Grundgerüst oder den konstanten Bereich des humanisierten Antikörpers nicht als fremd erkennen und daher sollte die Antikörper-Antwort gegen einen solchen injizierten Antikörper geringer ausfallen als gegen einen vollständig fremden Maus-Antikörper oder einen partiell fremden chimären Antikörper; (b) da der Effektorbereich des humanisierten Antikörpers menschlich ist, dürfte er mit anderen Teilen des menschlichen Immunsystems besser interagieren, und (c) injizierte humanisierte Antikörper weisen eine Halbwertszeit auf, die im wesentlichen zu der von natürlich vorkommenden menschlichen Antikörpern äquivalent ist, was es erlaubt, kleinere und weniger häufige Dosen im Vergleich zu Antikörpern anderer Spezies zu verabreichen.

In einer ganz besonders bevorzugten Ausführungsform ist die neutralisierende Verbindung eine lösliche Form eines LPS-assoziierten Rezeptors z.B. von CD14 und TLR-4, eines Komplement regulierenden Proteins z.B. von CD11b, CD18 und CD55, von Fcγ-R, z.B. von CD16a, CD32 und CD64, eines Integrin-assoziierten Proteins z.B. von CD81 und CD47, einen Scavenger Rezeptors z.B. CD36 und/oder eines G-Protein-Rezeptors.

Die vorstehend beschriebene neutralisierende Verbindung wird gegebenenfalls in Kombination mit einem pharmazeutisch verträglichen Träger verabreicht. Geeignete Träger und die Formulierung derartiger Arzneimittel sind dem Fachmann bekannt. Zu geeigneten Trägern zählen beispielsweise Phosphat-gepufferte Kochsalzlösungen, Wasser, Emulsionen, beispielsweise Öl/Wasser-Emulsionen, Netzmittel, sterile Lösungen etc. Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Erkrankung, der Art der Verabreichung, etc.

**Kurze Beschreibung der Figuren:**

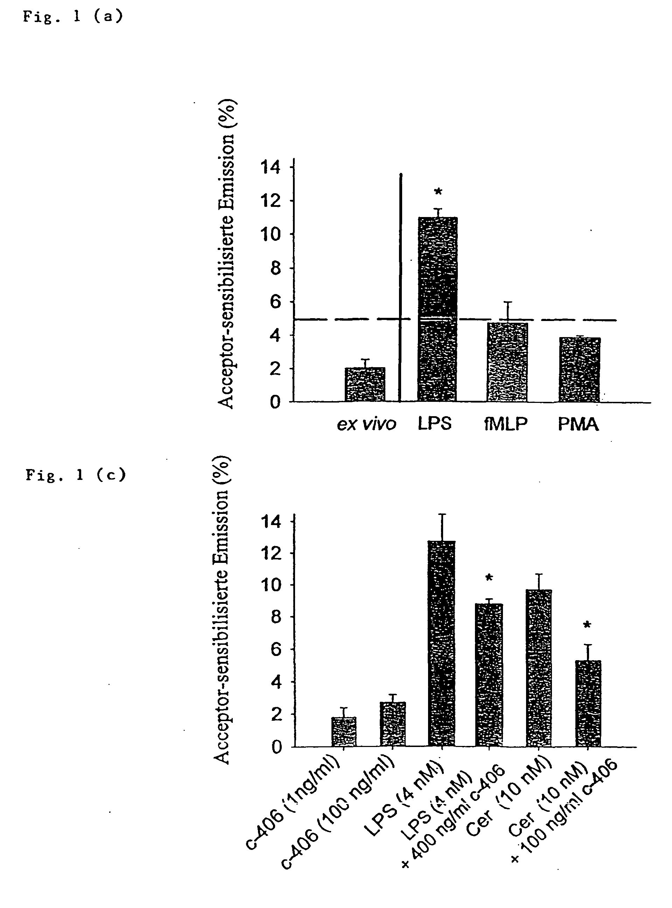

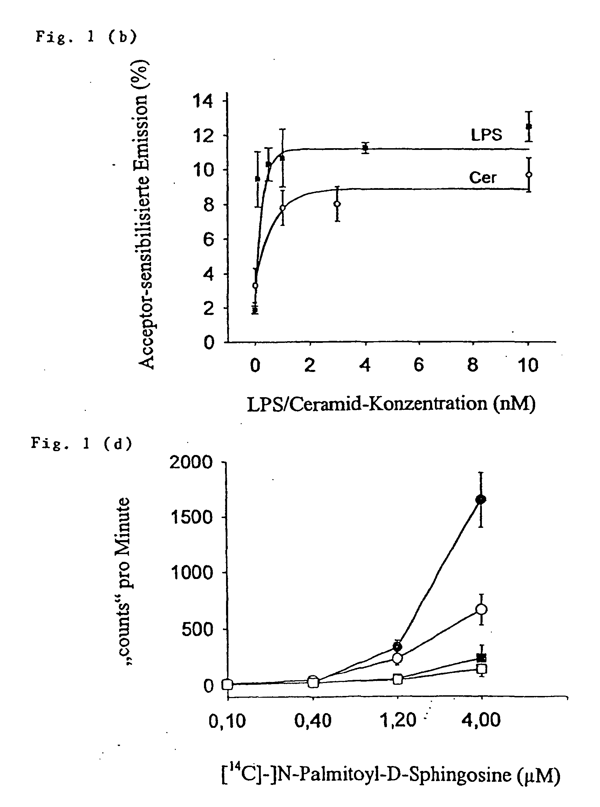

Figur 1: Liganden abhängige Konformationsänderung des CD14/CD11b Komplexes **a** Menschliche Monozyten wurden mit LPS (4 nM), fMLP und PMA (je 1 mM) oder mit PBS zur Kontrolle inkubiert und anschließend mit monoklonalen Antikörpern gegen CD11b oder CD14 markiert. Drei unterschiedliche Paare von Antikörpern wurden als Kontrolle untersucht, um die Spezifität des Energietransfers zu verifizieren. CD11b und CD18 zeigten einen starken Energietransfer auf ruhenden (ETp $\cong$ 60 % $\pm$ 2 %) und LPS-stimulierten Monozyten (ET$_p$ $\cong$ 65 % $\pm$ 2 %) und wurden als Positivkontrolle verwendet, während CD14 (Klone Uchm1 und x8) und CD33 keinerlei Ortsnähe aufwiesen und als Negativkontrolle fungierten (ET$_p$ < 5 %). Energietransferwerte sind in Einheiten der Emission Cy5, angeregt bei 488 nm (Acceptor-sensibilisierte Emission, Gleichung 1) und sind signifikant unterschiedlich von der Kontrolle mit p < 0.05 (*). **b** Konzentrationsabängiger Effect von LPS und Ceramid bezüglich der Ko-Assemblierung von CD11b und CD14. **c** Zellen wurden mit LPS (4 nM), Ceramid (10 nM), LA (1 ng/ml, 100 ng/ml), LPS (4 nM) + compound-406 (400 ng/ml, c-406) und Ceramid (10 nM) + c-406 (100 ng/ml) für 15 Minuten bei 37 °C inkubiert und mit monoklonalen Antikörpern markiert. Energietransfer Werte sind signifikant unterschiedlich im Vergleich zur Inkubation ohne den LPS-Antagonisten bei p < 0.05 (*). Ergebnisse repräsentieren den Mittelwert $\pm$ SD von 5 unabhängigen Experimenten. **d** CHO-Zellen transfizierte mit menschlichem CD14 (-unmarkiertes Ceramid (●), + unmarkiertes Ceramid (C)) oder mit einem pPOL-DHFR VeKtor (-unmarkiertes Ceramid (■), + unmarkiertes Ceramid (□)) wurden mit with den angegebenen Konzentrationen von [$^{14}$C]N-Palmitoyl-D-Sphingosine inkubiert und das an Zellen gebundene $^{14}$C wurde bestimmt. Jeder Datenpunkt repäsentiert den Mittelwert $\pm$ SD von dreifachen Messungen. Das Experiment wurde zweimal wiederholt mit vergleichbaren Ergebnissen.

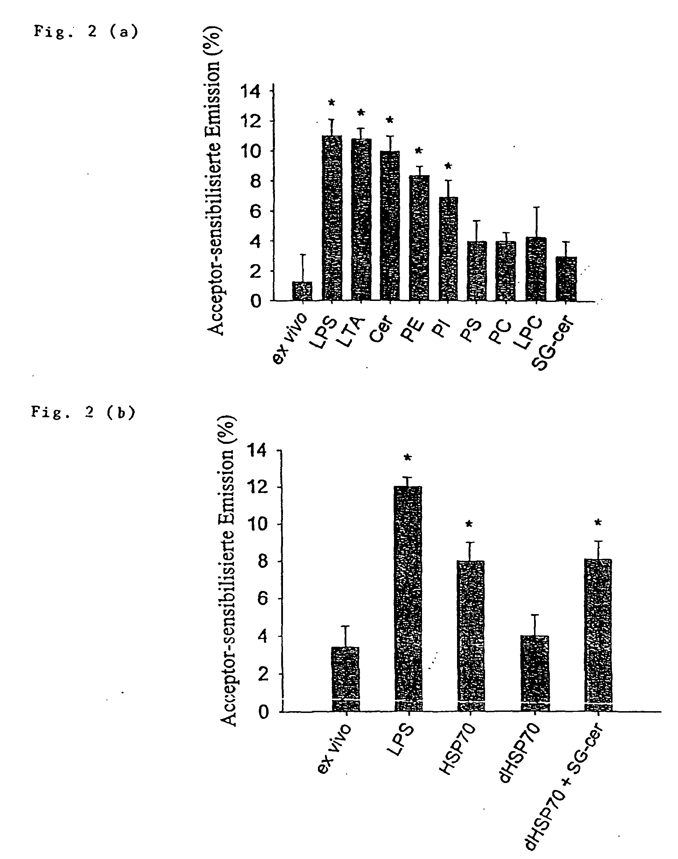

Figur 2: Alternative Agonisten induzieren die Co-Assemblierung von CD11b und CD14 **a** Die Effekte von LPS (4 nM), Lipoteichonsäure (1 mg/ml, LTA), Cer (40 nM), Phosphatidylethanolamin (10 mM, PE), Phosphatidylinositol (10 mM, PI) Phosphatidylserin (10 mM, PS), Phosphatidylcholin (10 mM, PC), Lyso-Phosphatidylcholin (10 mM, LPC) und Sulfogalactosylceramid (SG-cer, 40 nM) auf die Co-Assemblierung von CD11b und CD14 wurden mittels Akzeptor-sensibilisierter Emission (Gleichung 1) analysiert. **b** Die Effekte von LPS (4 nM), Heat shock protein 70 (7 nM, HSP70), delipidiertem HSP70 (7 nM, dHSP70) und SG-cer + dHSP70 (40 nM + 7 nM) auf die Co-assemblierung von CD11b und CD14 wurden mittels mittels Akzeptor-sensibilisierter Emission (Gleichung 1) analysiert. sind signifikant unterschiedlich von der Kontrolle mit p < 0.05 (*). p < 0.05 (*).Die Daten sind Mittelwerte $\pm$ SD von 10 unabhängigen Experimenten.

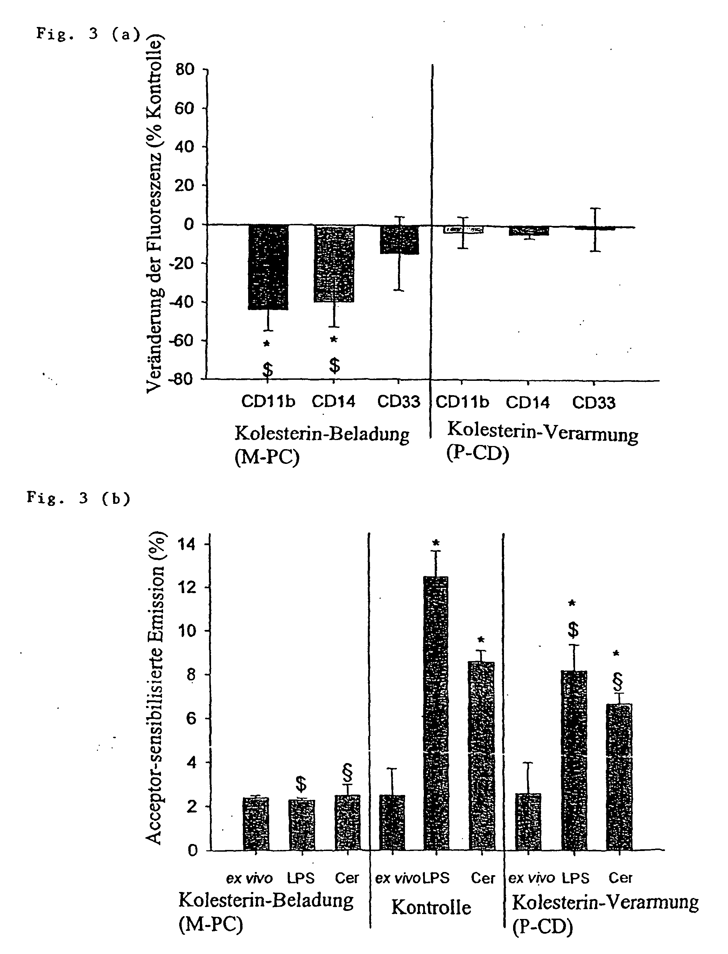

Figur 3: Auswirkung von Modifikationen in der "Raft"-Struktur auf den CD14/CD11b-Komplex

**(a)** Zellen wurden mit Methyl-Cyclodextrin/Cholesterin (4,6 mg/ml / 100 mg/ml, M-CD) für die Cholesterin-Beladung und Propyl-Cyclodextrin (0,15 mg/ml, P-CD) für die Cholesterin-Verarmung 30 Minuten bei 37°C inkubiert und außerdem mit monoklonalen CD14-PE-, CD33-PE- und CD11b-PE-Antikörpern markiert. Die Expressionsdichte der Rezeptoren wurde untersucht und - relativ zur unbehandelten Kontrolle (100%) - berechnet. Unterschiede in der Rezeptor-Expression im Vergleich zur Kontrolle waren bei p < 0,05 (*) signifikant und Unterschiede von CD11b und CD14 zu CD33 bei p < 0,05 ($). Die Daten sind Mittelwerte $\pm$ SD von 5 unabhängigen Experimenten.

**(b)** Menschliche Monozyten wurden mit Methyl-Cyclodextrin/Cholesterin (M-CD, 1 mg/ml / 22 mg/ml) und Propyl-Cyclodextrin (015 mg/ml, P-CD) 30 Minuten bei 37°C behandelt und weiter 15 Minuten bei 37°C mit LPS (4 nM), Ceramid (40 nM) und PBS inkubiert. Nach einem Waschschritt wurden diese mit den Antikörpern gegen CD11b und CD14 wie beschrieben markiert. Die Energietransfer-Werte sind als Akzeptor-sensibilisierte Emission (Gleichung 1) ausgedrückt. Die Energietransfer-Werte waren bei p < 0,05 (*) im Vergleich zur *ex vivo* Kontrolle signifikant und bei p < 0,05 im Vergleich zur Stimulierung mit LPS ($) oder Ceramid (§) ohne weitere Behandlung. Die Daten sind Mittelwerte $\pm$ SD von 5 unabhängigen Experimenten.

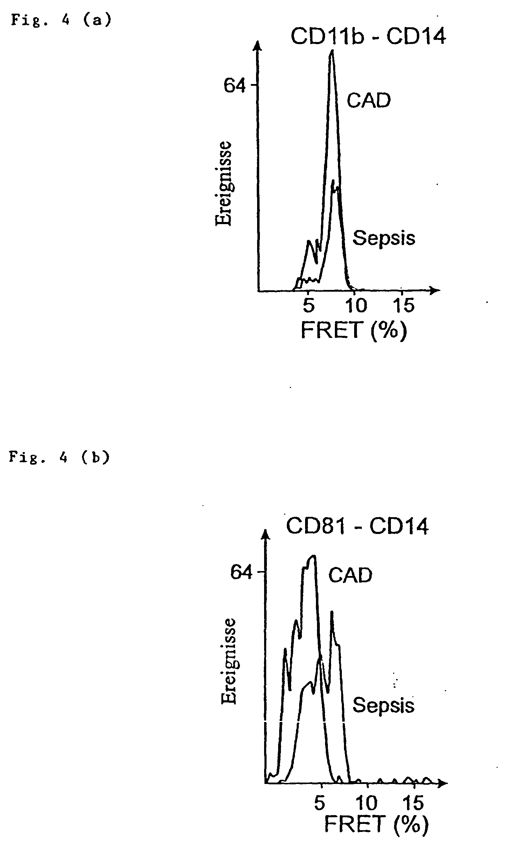

Figur 4: Analyse der Co-Assemblierung von Rezeptoren mit CD14 auf einzelnen Monozyten von Patienten mit Sepsis oder CAD
Ex vivo gewaschene Zellen von Patienten mit Sepsis und CAD wurden mit monoklonalen Antikörpern markiert. **(a)** Co-Assemblierung von CD11b und CD14 (b) Co-Assemblierung von CD81 und CD14. Energietransfer-Werte sind individuell für mehr als 300 Zellen (Sepsis) bzw. mehr als 600 Zellen (CAD) als durch Umwandlung gemäß Gleichung 1 erhaltenes Ein-Parameter-Histogramm angegeben.

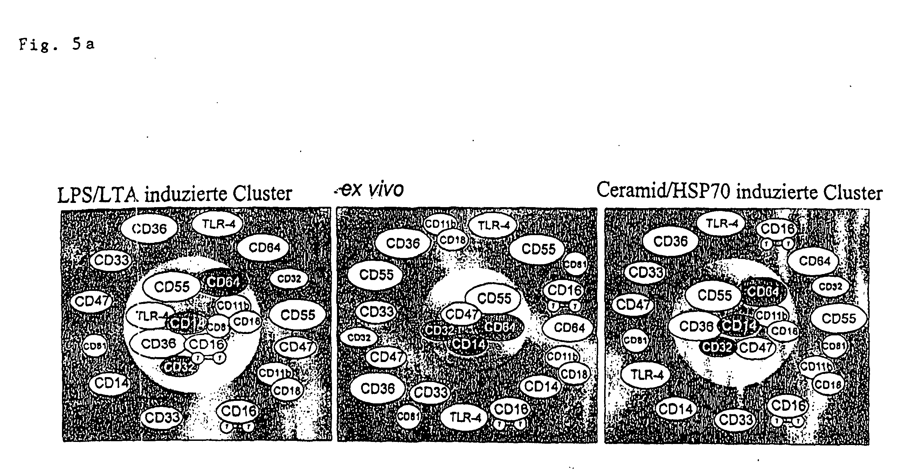

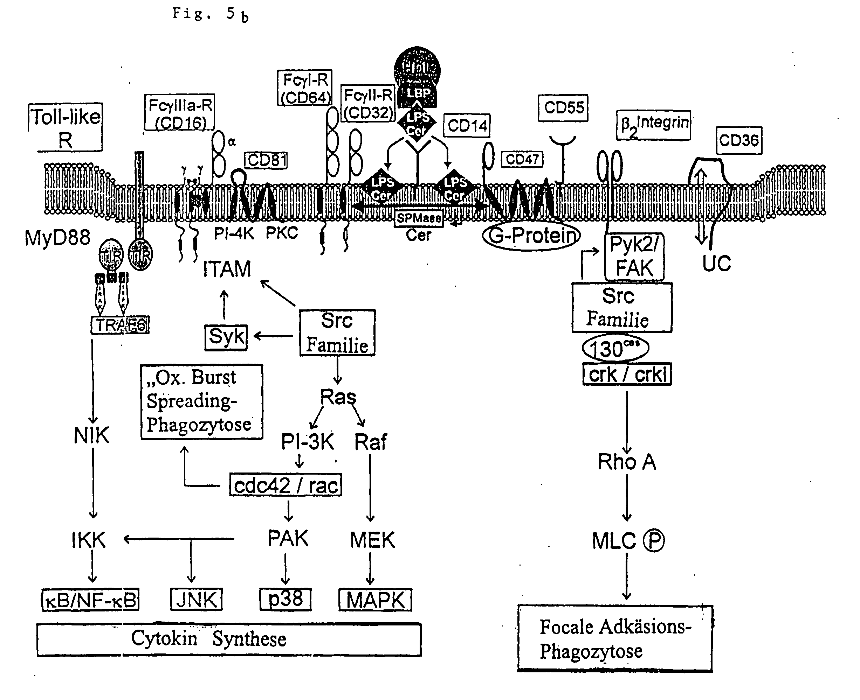

Figur 5: Rezeptor-Komplex bezüglich kongenitaler Immunität auf menschlichen Monozyten
Schematische Darstellung des durch LPS oder Ceramid induzierten Rezeptor-Komplexes auf menschlichen Monozyten. Eine Gruppe von Oberflächenrezeptoren ist an einem gemeinsamen Signaltransduktions-Komplex beteiligt, der bei der Kontrolle der Rezeptorgenexpression, der Wachstumsregulation und der Apoptose einer Entzündungsreaktion und Reorganisation des Zytoskeletts eine wichtige Rolle spielt.

Die folgenden Beispiele erläutern die Erfindung.

**Beispiel 1: Allgemeine Verfahren**

(A) Blutproben

Proben von heparinisiertem peripheren Blut wurden von gesunden männlichen und weiblichen Blutspendern erhalten. Diese Spender waren frei von Erkrankungen oder Infektionen, was durch entsprechende Untersuchungen nachgewiesen worden war, und nahmen zumindest innerhalb der letzten 14 Tage vor Blutentnahme keine Medikamente ein. Blutproben wurden auch von Patienten mit Sepsis, einer Erkrankung der Koronararterien (CAD), definiert mittels Koronar-Angiographie, entweder einem akuten Myokardinfarkt oder Angina Pectoris, oder mit einem akuten Schlaganfall erhalten. Dies erfolgte mit Zustimmung der Patienten und des örtlichen Ethik-Kommittees (Nr. 00/33). Die Blutproben aller Patienten wurden bezüglich LPS in einem "chromogenic limulus amebocyte lysate (LAL) assay" (QCL-100, Biowhittaker, Walkersville, USA) untersucht, wobei die Hälfte der Patienten mit Sepsis einen positiven Befund zeigte, während alle anderen Proben negativ hinsichtlich LPS waren.

(B) Herstellung von Lipoteichonsäure

Bacillus subtilis wurde in einer 8 L-Schüttelkultur gezüchtet. Die Bakterien wurden auf Eis mittels Ultraschallbehandlung (Branson-"Sonifier", Branson, Schwäbisch-Gmünd, Deutschland) aufgebrochen und bei Raumtemperatur Butanol-extrahiert. Die wäßrige Phase wurde mittels auf hydrophoben Interaktionen beruhender Chromatographie (HIC) auf Octylsepharose (Fischer, Anal. Biochem. 208 (1993), 49-56; Fischer, Anal. Biochem. 194 (1991), 353-358; Fischer, Eur. J. Biochem. 133 (1983), 523-530; Leopold und Fischer, Anal. Biochem. 201 (1992), 350-355) gereinigt. Die Fraktionen wurden bezüglich Phosphor-reicher Lipoteichonsäure mittels des Phosphomolybdenblau-Assays (Schnittker et al., Biochemische Zeitschrift (2000), 167-185) gescreent. Alle Lipoteichonsäure-Präparationen erwiesen sich im LAL-Assay als LPS-negativ.

(C) Delipidierung von HSP70

Heat shock protein 70 (HSP70) wurde von Stressgen (Victoria, Canada) bezogen. 200 g HSP70 wurden in Ethanol/Diethylether (3:1) bei - 20 °C über Nacht delipidiert. Nach einem Zentrifugationsschritt (15 Minute bei 4000 U/min) wurde das erhaltene Pellet in Diethylether bei - 20 °C für 4 Stunden delipidiert. Nach Zentrifugation (15 Minuten, 4000 U/min) wurde das delipidierte HSP70 (dHSP70) in 50 nM Tris-HCl pH 7.5,100 mM NaCl, 1 mM Dithiothreitol und 0.1 mM Phenylmethylsulfonylfluorid gelöst. HSP70 war LPS-negativ im the LAL-Assay.

(D) Herstellung von Phospholipid-Liposomen

Phosphatylethanolamin (P), Posphatidylcholin (PC), Phosphatidylinosit (PI) und Phosphatidylserin (PS) wurden von Sigma (Deisenhofen, Deutschland) als Chloroform-Lösungen bezogen. Die Lipide wurden in einem Vakuum-Rotationsverdampfer getrocknet und in Dulbecco-modifizierter Phosphatpuffer-Kochsalzlösung ohne $Ca^{2+}$ und $Mg^{2+}$ (PBS) mittels Ultraschallbehandlung resuspendiert. Der Lipidgehalt wurde mittels Dünnschichtchromatographie bestimmt. Alle Liposomen-Präparationen erwiesen sich im LAL-Assay als LPS-negativ.

(E) Stimulierung von Blutproben

Aliquots (100 ml) von Vollblut wurden 15 Minuten bei 37°C mit den folgenden stimulatorischen Verbindungen inkubiert: LPS von Salmonella minnesota (0,1 - 10 nM, MG = 10 kDa), Ceramid (hauptsächlich Stearinsäure und Nervonsäure) und Sulfogalactosylceramid (SG-cer, 40 nM), beidegemäß LAL-Assay LPS-frei (Cer, 1 nM - 0,5 mM), Lysophosphatidylcholin (lyso-PC, 10 mM), Phorbol-12-myristat-13-acetat (PMA, 1 mM), N-Formyl-L-methionyl-L-leucyl-L-phenylalanin (fMLP, 1 mM), PE-, PC-, PI- und PS-Liposomen (10 mm), die wie vorstehend beschrieben hergestellt wurden. Alle Reagenzien wurden von Sigma bezogen. Außerdem wurden die Zellen mit HPS70 (7 nM), dHSP70 (7 nM) und SG-cer + dHSP70 (40 nM + 7 nM) stimuliert. Nach Inkubation wurden die Zellen mit kaltem, 0,1% $NaN_3$ enthaltendem PBS gewaschen. Der synthetische LPS-Antagonist Lipid IVa ("compound-406"; 1-400 ng/ml) und von E. coli stammendes Lipid A (1-100 ng/ml) wurden freundlicherweise von Dr. S. Kusumoto zur Verfügung gestellt. Die Zellen wurden 15 Minuten bei 37° mit dem LPS-Antagonisten vorinkubiert und danach mit LPS bei der gleichen Temperatur (ohne Waschen) stimuliert.

(F) Bindung von [$^{14}$C]N-Palmitoyl-D-Sphingosin an CHO-Zellen

Mit humanem CD14 oder dem pPOL-DHFR-Vektor transfizierte Zellen wurden wie kürzlich beschrieben gezüchtet (Stelter et al., Eur. J. Biochem. 238 (1996), 457-464). Die Zellen wurden trypsiniert, zweimal mit PBS gewaschen und dann in 10% menschliches Serum enthaltender PBS aufgenommen. CHO-Zellen (4x10$^5$) wurden auf Eis in Gegenwart eines 50fachen Überschusses von unmarkiertem N-Palmitoyl-D-Sphingosin (Sigma) 15 Minuten in einem Endvolumen von 100 ml vorinkubiert. Danach wurde [$^{14}$C]N-Palmitoyl-D-Sphingosin (American Radiolabeled Chemicals Inc., St. Louis, MO, USA) zugegeben und die Zellen wurden weitere 15 Minuten inkubiert. Zur Entfernung nichtgebundener Aktivität wurden die Zellen zweimal gewaschen und dann in ein frisches Eppendorf-Gefäß überführt. Nach erneutem Waschen wurde das Pellet in 100 ml 1 % SDS, 10 mM EDTA gelöst und danach zu 3 ml Szintillationsflüssigkeit gegeben. Die Menge an Zell-gebundener Radioaktivität wurde mittels Flüssigkeitsszintillationsmessung bestimmt.

(G) Immunfärbung von stimulierten Zellen

Stimulierte oder ex vivo gewaschene Vollblutproben (200 ml) wurden 15 Minuten auf Eis mit Sättigungskonzentrationen von monoklonalen Fluorochrom-konjugierten oder biotinylierten Antikörpern inkubiert. Die monoklonalen Antikörper CD11a (Klon 25.3.1), CD16a (Klon 3G8), CD18 (Klon 7E4), CD14 (Klon RMO52), CD33 (Klon My9-RD1) CD81 (Klon Js64) wurden als R-Phycoerythrin(R-PE)-Konjugate und CD25 (Klon B1.49.9) in biotinylierter Form Coulter/Immunotech (Krefeld, Deutschland) erhalten. CD11b (Klon d12), CD11c (Klon S-HCL-3), CD36 (Klon CB38), CD47 (Klon B6H12), CD81 (Klon JS-81) und CD55 (Klon IA10) in biotinylierter Form und Streptavidin-R-PE wurden von Becton Dickinson/Pharmingen (Heidelberg, Deutschland) bezogen. CD18 (Klon IB4) in biotinylierter Form und CD40 (Klon EA-5) als R-PE-Konjugat wurden von Alexis (Grünberg, Deutschland bezogen und CD14 (Klone x8 und Uchm1) und CD3 (Klon Cris-7) in biotinylierter Form und Streptavidin-Cy5 (SA-Cy5) von Dianova (Hamburg, Deutschland). CD32 (Klon C1 KM5) und CD64 (Klon 10.1) wurden als R-PE-Konjugate von Caltag (Burlingame, USA) bezogen und CD91 (Klon 8G1) in biotinylierter Form von Progen (Heidelberg, Deutschland). Ein monoklonaler anti-TLRA-Antikörper (Klon HTA125) wurde freundlicherweise von Dr. Kensuke zur Verfügung gestellt. Da zwischen den gegen die CD14/CD11a- und CD14/CD11c-Paare gerichteten Antikörper sowohl auf ruhenden als auch stimulierten Zellen kein Energietransfer nachweisbar war, wurden diese Antikörper-Kombinationen nicht weiter charakterisiert.

Die Lyse von Erythrozyten und das Waschen wurden wie beschrieben durchgeführt (Rothe et al., Thromb. Vasc. Biol. 16 (1996), 1437-1447). Nach dem letzten Waschschritt wurden die Proben geteilt und ein Teil jeder Probe wurde mit einer Sättigungsmenge an SA-Cy5 15 Minuten auf Eis inkubiert und vor der Messung gewaschen.

(H) Modifikationen der Raft-Struktur

Mononucleäre Zellen wurden mittels "Histopaque 1077"-Dichtegradientenzentrifugation (Sigma) aus Proben heparinisierten Bluts isoliert. Als wirksamer Donor für die Cholesterin-Beladung der Zellmembran wurde methylisiertes β-Cyclodextrin (Sigma) verwendet. Aliquots mononukleärer Zellen (5x10$^5$ Zellen/ml) wurden in 3 ml PBS resuspendiert und 30 Minuten bei 37°C mit Komplexen aus Cholesterin (100 mg/ml) und Methyl-β-Cyclodextrin (4,6 mg7ml) inkubiert, die wie in Christian et al., J. Lipid. Res. 38 (1997), 2264-2272) beschrieben für die Cholesterin-Beladung hergestellt wurden. Die Zellen wurden zur Cholesterin-Verarmung mit 2-Hydroxy-propyl-β-cyclodextrin (Sigma, 0,15 mg/ml) 30 Minuten bei 37°C inkubiert. Nach Inkubation mit Cyclodextrinen wurden die Zellen gewaschen, wie vorstehend beschrieben immungefärbt und die Expression der Antigene wurde mittels Durchflußzytometrie gemessen.

Für Energietransfer-Experimente wurden 200 ml-Aliquots von Vollblut 30 Minuten bei 37°C mit Cholesterin/Methyl-Cyclodextrin (C/C) (100 mg/ml / 4,6 mg/ml) oder 2-Hydroxypropyl-β-Cyclodextrin (0,15 mg/ml) behandelt und dann mit LPS (4 nM) oder Ceramid (40 nM) wie beschrieben weiter stimuliert. Der Energietransfer zwischen CD14 und CD11b wurde gemessen.

(I) Messung des FRET mittels Durchflußzytometrie

Es wurde ein "FACSCalibur"-Durchflußzytometer (Becton Dickinson) verwendet, das mit einem Luft-gekühlten 15 mW Argonlaser (Anregungswellenlänge 488 nm), einem Dioden-Laser (Anregungswellenlänge 625 nm) und Standardfiltern (Kanal 1 (FITC): 530/30 nm Bandpassfilter; Kanal 2 (R-PE wurde als Donor-Farbstoff verwendet): 585/47 nm Bandpassfilter; Kanal 3 (PerCP): > 670 nm langer Pass; und Kanal 4 (Cy5 wurde als Empfänger-Farbstoff verwendet): 661/16 nm Bandpassfilter) versehen war. Für die Messung wurde die Software Cellquest (Becton Dickinson) verwendet. Die Photomultiplier(PMT)-Spannung wurde für alle Fluoreszenzkanäle eingestellt, was eine mittlere Autofluoreszenz der ungefärbten Leukozyten im Zentrum der ersten der vier Log-Ordnungen ergab. Die Messungen wurden ohne Kompensation durchgeführt. Im "list"-Modus waren 50.000 Leukozyten erforderlich, wobei Vorwärtsstreuung (FSC) als Trigger-Signal verwendet wurde.

Die Leukozyten wurden zuerst in einen FSC versus SSC (Seitenstreuung) Dot-Plott wie bereits beschrieben (Rothe et al., Thromb. Vasc. Biol. 16 (1996), 1437-1447) geleitet. Ein zweites auf der Donor-Antikörper-Expression basierendes Tor (Kanal 2) wurde zur Trennung der Mikropartikel, Debris oder Aggregate verwendet. Die mittleren Fluoreszenzintensitätswerte wurden für die Kanäle 2,3 und 4 berechnet.

(J) Bestimmung der FRET-Effizienz (Akzeptor-sensibilisierte Emission oder Donor-Auslöschung)

Mittels des FRET können Entfernungen zwischen Öberflächenmolekülen gemessen werden (Szollosi et al., Cytometry 34 (1998), 159-179). FRET ist ein nicht auf Strahlung beruhender Energietransfer von einem angeregten Donorfluorophor zu einem Akzeptorfluorophor über Dipol/Dipol-Interaktionen. Die Berechnungen wurden gemäß einer vereinfachten Version des von Szollosi et al., Supra, beschriebenen Verfahrens durchgeführt. Der Energietransfer-Parameter ($Et_p$), der zu der FRET-Effizienz (ET) proportional ist, wurde gemäß Formel (1) berechnet, wobei A der Akzeptor ist, D der Donor, FL2 die mittlere Fluoreszenz in Kanal 2 (Donor), FL3 die mittlere Fluoreszenz in Kanal 3 und FL4 die mittlere Fluoreszenz in Kanal 4 (Akzeptor) (wobei jeder Wert nach Subtraktion der Autofluoreszenz erhalten wurde):

$$ET_p = \frac{FL3(D,A)-FL2(D,A)/a-FL4(DA)/b}{FL3(D,A)} \tag{1}$$

$$a=FL2(A)/FL3(A)$$

$$b=FT4(D)/FL3(D)$$

Die FRET-Effizienz (ET) wurde gemäß Formel (2) als Auslöschung berechnet:

$$ET = \frac{FL2(D)-FL2(D,A)}{FL2(D)} \tag{2}$$

Gemäß der Veröffentlichung von Szollosi et al. (Cytometry 5 (1984), 210-216) wurde in einleitenden Experimenten eine ET=5% als das Schwellenniveau für eine signifikante Transfereffizienz definiert. Dieses Schwellenniveau wurde experimentell als die zu dem $Et_p$-Wert proportionale Nachweisgrenze bestätigt. Bei allen späteren Messungen wurden eine ET > 5% oder ein $ET_p$ > 5% als positiver Transfer angesehen. Bei einigen Messungen gab es eine Diskrepanz zwischen dem von der Donor-Auslöschung und dem von der sensibilisierten Emission berechneten Energietransfer, was allerdings durch eine Verdrängung des Donors durch den Akzeptor erklärt werden kann. Es wurde somit nur die Auslöschung berechnet, die von diesem Phänomen nicht betroffen ist (Lakowicz, In: Principles of fluorescence spectroscopy, 305-341; Plenum Press, New York, 2000).

(K) Messung der Ceramid-Spiegel im Plasma

Plasma-Ceramid-Spiegel wurden wie kürzlich beschrieben (Liebisch et al., J. Lipid. Res. 40 (1999), 1539-1546) quantifiziert. Dazu wurden Plasmaproben mit C8-Ceramid (Sigma) gespiked und gemäß dem Verfahren von Bligh und Dyer (Ca. J. Biochem. Physiol. 37 (1959), 911-917) extrahiert. Die Quantifizierung wurde mittels einer Kalibrierungskurve ermöglicht, die durch das "Spiking" mit unterschiedlichen Konzentrationen natürlich vorkommender Ceramid erstellt worden war. Die Messungen wurden mit einem "API 365 triple quadrupol"-System (Perkin Elmer, Wellesley, USA) durchgeführt, das mit einem "turbo ion spray"-Interface ausgestattet war.

(L) Statistische Analysen

Die Daten sind als Mittelwerte ± Standardabweichung (SD) angegeben. Zum statistischen Vergleich zwischen Kontroll- und Patientengruppen wurde der Student-t Test unter Verwendung der SPSS-Software (SPSS Inc, Chicago, USA) durchgeführt, zum Vergleich zwischen Kontroll-Proben und *in vitro* stimulierten Proben der gepaarte Student-t Test.

**Beispiel 2: Liganden-induziertes Clustern von CD11b und CD14 in"Rafts"**

Es wurden zuerst Bedingungen untersucht, unter denen die Co-Assemblierung dieser beiden Rezeptoren auf Monozyten induziert wird. Menschliche Monozyten wurden in *vitro* mit LPS, fMLP und PMA stimuliert und der Energietransfer zwischen monoklonalen Antikörpern gegen CD11 b und CD14 wurde gemessen. Die Ergebnisse zeigen, daß die Co-Assemblierung von CD14 und CD11 b in einem Prozeß der konformationellen Aktivierung allein durch LPS induziert wird (Figur 1a). Auf ruhenden Monozyten wurde zwischen monoklonalen Antikörpern gegen CD14 (Klone x8 oder Uchm1) und CD11b kein signifikanter Energietransfer beobachtet (Figur 1a). Auf LPS-stimulierten Monozyten wurden zwischen anti-CD11b- und anti-CD14-Antikörpem ein Energietransfer beobachtet, was ein Anzeichnen für ein Aktivierungs-abhängiges Clustern dieser beiden Moleküle ist: Der Effekt war Dosis-abhängig und Sättigung wurde bei etwa 1 nM LPS erreicht. Das Clustern war LPS-spezfisch, da weder fMLP noch PMA eine Wirkung zeigten (Figur 1 a). Synthetisches Lipid A induzierte, ähnlich wie LPS, die Co-Assemblierung von CD11b und CD14.

Da bekannt ist, daß Ceramid die LPS-induzierte Antwort nachahmen kann, wurde die Auswirkung von Ceramid auf die Co-Assemblierung von CD11b und CD14 untersucht. Figur 1 b zeigt den Ceramid-induzierten Energietransfer zwischen Antikörpern gegen CD11b und CD14 (Klon x8) auf menschlichen Monozyten. Die $EC50_{Cer}$ war im Bereich von 0,1 nM, was mit LPS vergleichbar ist. Diese Ergebnisse zeigen, daß Ceramid die Überführung von CD14 in ein Rezeptor-Cluster induziert. Um zu untersuchen, ob Ceramid mit CD14 über einen ähnlichen Mechanismus wie LPS interagiert, wurde vor der Stimulierung der Zellen mit beiden Agonisten ein potenter LPS-Antagonist (synthetisches Lipid IVa, "compound-406") verabreicht und das Clustern von CD11b und CD14 wurde dann untersucht. Die Verabreichung von "compound-406" allein (1 und 100 ng/ml) führte nicht zu einer Induktion der Co-Assemblierung der Rezeptoren CD14 und CD11b (Figur 1c), die Verabreichung von Lipid IVa *in vitro* 15 Minuten vor der Stimulierung verringerte jedoch die LPS- oder Ceramid-induzierte Co-Assoziation zwischen CD14 und CD11b signifikant (Figur 1c).

Ein weiteres Anzeichen für eine spezifische Interaktion von Ceramid mit CD14 war die Bindung von [$^{14}$C] -N-Palmitoyl-D-Sphingosin (C16-Ceramid) an CD14-transfizierte CHO-Fibroblasten, die im Vergleich zu Vektor-transfizierten Zellen viel höher war und die durch die Zugabe eines 50fachen Überschusses von unmarkiertem Liganden gehemmt werden konnte (Figur 1 d). Diese Ergebnisse zeigen, daß Ceramid ein extrazellulärer Ligand von CD14 ist.

Zur weiteren Untersuchung von Liganden, die die Co-Assemblierung von CD14 induzieren, wurden Monozyten mit Lipoteichonsäure, SG-cer und verschiedenen Glycero-Phospholipid-Liposomen inkubiert, zu denen PI, PS, PE, lysoPC und PC zählten. Lediglich Lipoteichonsäure, PE und PI induzierten eine Co-Assemblierung zwischen CD11b und CD14 (Figur 2 a). Außerdem zeigte sich, daß natürliches HSP70 ebenfalls ein Clustering von CD11b und CD14 auslöst, delipidiertes HSP70 (dHSP70) hingegen induziert keinen Rezeptor-Komplex. Nachdem bekannt ist, daß HSP70 and SG-cer bindet, wurde dHSP70 mit SG-cer rekombiniert. Im Gegensatz zu SG-cer alleine (Figur 2 a) und lipidfreiem dHSP70 (dHSP70) induzierten SG-cer/dHSP70-Komplexe eine Assemblierung von CD11 b und CD14 (Figur 2 b), was auf eher auf eine Lipid-Protein-vermittelte als eine Protein-Protein-vermittelte Wechselwirkung von HSP70 und CD14 hideutet.

Es konnte in verschiedenen zellulären Modellen gezeigt werden, daß CD14 mit "Rafts" assoziiert ist. Daher wurde die Co-Assemblierung von CD11b und CD14 in Abhängigkeit von der Modifizierung der Cholesterin-Zusammensetzung der Plasmamembran mit Cyclodextrinen untersucht, um so sowohl die "Raft"-Assoziation als auch die regulatorische Rolle der "Raft"-Lipide auf die Funktion von CD14 bei menschlichen Monozyten nachweisen zu können. Die Cholesterin-Beladung der Membran für 30 Minuten führte zu einer Abnahme der CD14- und CD11b-Expression (Figur 3a) und hemmte die LPS-induzierte Co-Assemblierung von CD14 und CD11b (Figur 3b) völlig. Im Gegensatz dazu hatte die Cholesterin-Verarmung der Membran keine Auswirkung auf die Expressionsdichte von CD14 und CD11b (Figur 3a). Die Co-Assoziierung von beiden Rezeptoren nach Stimulierung mit LPS war jedoch signifikant verringert (Figur 3b). Analoge Messungen zeigten, daß das Ceramid-induzierte Cluster ähnlich beeinflußt wurde (Figur 3). Dies zeigt, daß eine intakte "Raft"-Zusammensetzung eine regulatorische Rolle bei der Bildung der Rezeptor-Cluster spielt.

**Beispiel 3: Charakterisierung der Oberflächenrezeptoren in der Nachbarschaft von CD14**

Es wurde untersucht, ob weitere Membranproteine nach LPS- oder Ceramid-Stimulierung mit CD14 sterisch assoziiert sind. Dazu wurde der Energietransfer unter Verwendung einer Reihe von Paaren von monklonalen Antikörpern analysiert. Es zeigte sich, daß in nicht-stimulierten Zellen CD14 mit den Fcy-Rezeptoren (Fcy-R) CD32 und CD64, dem Komplement-regulierenden Protein CD55 und mit dem Integrin-assoziierten Protein (IAP) CD47 geclustert ist, während das Clustern mit den Komplement-regulierenden Proteinen CD11b, CD18, dem LPS-Signalüberträger TLR-4, dem Fcy-R CD16a, dem "scavenger"-Rezeptor CD36 und dem IAP CD81 nur nach LPS-Stimulierung nachweisbar war (Tabelle 1a und 1b, Figur 5). Im Gegensatz dazu war CD47 nach LPS-Stimulierung nicht mehr mit CD14 assoziiert. Diese Befunde zeigen, daß die LPS-Stimulierung die Clusterbildung von Rezeptoren auf der Monozyten-Oberfläche induziert, wobei zu den Mitgliedern dieser Cluster CD14, die Komplement-regulierenden Proteine, TLR4, die Fcy-Rezeptoren, CD18 und der "scavenger"-Rezeptor CD36 zählen. LTA induziert ein identisches Cluster im Vergleich zum LPS-induziertem Komplex.

In Ceramid-stimulierten Monozyten waren - im Gegensatz zu LPS-stimulierten Zellen - CD16a, TLR-4 und CD81 nicht mit CD14 assoziiert, wobei jedoch CD47 noch mit CD14 assoziiert war (Tabelle 1a und 1b, Figur 5). Somit unterscheidet sich das Ceramid-induzierte Cluster von dem LPS-induzierten hinsichtlich der integrierten Mitglieder. CD14, Komplement-regulierende Proteine, CD11b, CD18, CD55, IAP CD47, Fcg-R CD32, CD64 und der "scavenger"-Rezeptor CD36. SG-cer/HSP70 wiederum induziert ein Cluster, das dem Ceramid-induziertem entspricht. Somit bringen LPS (LTA) und Ceramid (HSP70) nach Bindung an CD14 unterschiedliche Rezeptoren in ein Cluster.

Tabelle 1

| Co-Assemblierung von Monozyten-Rezeptoren mit CD14 (Akzeptor- sensibilisierte Emission (%) (a) und Donor-Auslöschung (%) (b) | | | | | |
|---|---|---|---|---|---|
| a | *ex vivo* | LPS | Cer | LTA | HSP70 |
| CD18(7E4)/ CD14(x8) | 2.4 ± 2.1 | 9.3 ± 1.1 * | 5.4 ± 0.8 * | 6.0 ± 2.1 * | 6.0 ± 2.1 * |
| CD36/CD14 (x8) | 3.8 ± 0.8 | 9.9 ± 0.9 * | 10.1 ± 1.0 * | 6.7 ± 0.9 * | 8.2 ± 1.8 * |
| CD55/CD14 (Uchm1) | 6.6 ± 2.5 | 8.5 ± 2.0 | 9.7 ± 1.5 | 7.2 ± 2.0 | 6.0 ± 0.5 |
| CD81(Js64)/ CD14(x8) | 1.8 ± 0.5 | 7.3 ± 0.5 * | 2.8 ±0.7 | 6.0 ± 1.4 * | 3.8 ± 2.5 |
| CD81(JS-81)/ CD14(x8) | 2.1 ± 0.5 | 6.7 ± 0.1 * | 3.1 ± 2.4 | 5.7 ± 0.1 * | 1.0 ± 0.1 |
| TLR-4/CD14 (x8) | 2.2 ± 0.6 | 9.1 ± 0.6 * | 3.2±2.5 | 7.8 ± 2.3 * | 5.4 ± 0.6 |
| CD32/CD14 (Uchml) > | 7.8 ±1.5 | 19.6 ±3.5 | 11.6 ±3.4 | 6.0 ±2.0 | 5.3 ± 2.1 |
| CD64/CD14 (Uchm1) | 9.4 ± 2.5 | 10.0 ±2.2 | 10.4 ± 1.3 | 7.5 ± 1.8 | 10.1 ± 1.5 |
| CD47/CD14 (Uchm1) | 10.5 ±1.0 | 4.0 ± 0.9 * | 11.9 ± 3.1 | 5.8 ± 0.9 * | |
| CD16/CD14 (Uchm1) | 0.5 ± 2.4 | 24.1 ± 4.2 * | 3.2 ± 5.6 | 35.7 ± 3.2 * | |

Zellen wurden mit LPS (4 nM), Ceramid (40 nM), LTA 8 1 µg/ml) und HSP70 (7 nM) inkubiert und mit monoklonalen Antikörpern wie in Beispiel 1 beschrieben markiert. Klone monoklonaler Antikörper wurden auf der Basis des höchsten Transfers nach Stimulierung mit LPS ausgewählt. Energietransfer-Werte sind entsprechend der Emission von Cy5 (angeregt bei 488 nm; Akzeptor-sensibilisierte Emission, Formel 1) ausgedrückt. Als Kontrolle für unspezifische Fc-Rezeptor-Bindung wurde der Energietransfer zwischen CD16, CD32, CD64 und zwei nicht-monozytären monoklonalen Antikörpern (CD3 und Vß8) oder zwei monozytären Antigenen (CD25 und CD91) gemessen. Die Stimulierung der Zellen mit fMLP oder PMA (als Kontrolle) führte zwischen keinem Antikörperpaar zu einem signifikanten Energietransfer. Die Energietransfer-Werte sind entsprehend der Auslöschung der Donor-Fluoreszenz ausgedrückt (Formel 2). Energietransfer-Werte waren bei $p < 0,05$

(*) signifikant. Die Daten sind Mittelwerte ± SD von 10 unabhängigen Experimenten.

## Beispiel 4: Co-Assemblierung von Monozyten-Rezeptoren mit CD14 bei mit Entzündungen einhergehenden Erkrankungen

Sepsis ist oft mit der akuten Freisetzung von LPS und LPS-vermittelter zellulärer Aktivierung assoziiert, während es sich bei Erkrankungen der Koronararterien (CAD) und Schlaganfall um Erkrankungen mit einer chronischen entzündlichen Monozyten/Gefäßwand-Interaktion handelt. Es wurden daher in der vorliegenden Erfindung Energietransfer-Messungen an Monozyten von solchen Patienten durchgeführt, um untersuchen zu können, ob auch *in vivo* eine ähnliche Co-Assemblierung von Rezeptoren induziert wird. Patientenproben wurden hinsichtlich der Rezeptorkonformation auf ruhenden (*ex vivo*) und *in vitro* LPS-stimulierten Monozyten im Vergleich zu Blutproben von gesunden Spendern untersucht (Tabelle 2a). Monozyten von allen Patientengruppen mit mit Entzündungen einhergehenden Erkrankungen jedoch nicht die Kontrollgruppe zeigten einen Energietransfer zwischen CD11b und CD14 bereits *ex vivo* vor der zusätzlichen Stimulierung *in vitro.* Die Anwesenheit von LPS bei Sepsis könnte die Co-Assemblierung von CD11 b/CD14 in dieser Patientengruppe erklären, nicht jedoch in den anderen Patientengruppen. Da Ceramid auch *in vitro* ein Clustern von CD11 b/CD14 induzieren konnte (Figur 1 b) und frühere Studien von atherogenen Lipoproteinen über erhöhte Ceramid-Spiegel berichteten, wurden die Ceramid-Spiegel im Plasma mittels Tandem-Massenspektroskopie ermittelt. Signifikant veränderte Plasma-Spiegel unterschiedlicher Ceramid-Spezies (C22, C23, C24:0 und/oder C24:1) wurden bei Patienten mit CAD, Schlaganfall und Sepsis gefunden (Tabelle 2b). Nur C24:1 war jedoch in allen Patientengruppen erhöht. Bei Sepsis zeigte sich die stärkste Veränderung hinsichtlich der Konzentrationen der unterschiedlichen Ceramid-Spezies: Die Spiegel von C24:1 und C22 waren erhöht, während die von C24:0 und C23 verringert waren.

Da Ceramide für die Aktivierung der CD14/CD11b-Cluster *in vivo* verantwortlich sein könnten und da *in vitro* die Bildung unterschiedlicher Cluster nach Induktion mit LPS bzw. Ceramid beobachtet werden konnte, wurden die Cluster bei Patienten mit Sepsis und CAD detaillierter untersucht. Die Co-Assemblierung von CD11 b und CD14 wurde in allen Patientengruppen beobachtet (Figur 4a). Die Co-Assemblierung von CD81 und CD14 wurde jedoch nur bei Patienten mit Sepsis beobachtet, bei CAD-Patienten konnte kein Energietransfer nachgewiesen werden (Figur 4b). Diese Ergebnisse zeigen, daß CD14 zusammen mit begleitenden, von unterschiedlichen Liganden abhängigen Proteinen zu einem spezifischen kongenitalen Rezeptorkomplex innerhalb von "Rafts" assembliert und so eine Signalgebung während nicht-adaptiven Immunreaktionen vermittelt.

Tabelle 2

| (a) Co-Assemblierung von CD11b und CD14, (b) Plasma-Ceramidspiegel bei Patienten mit CAD, Schlaganfall und Sepsis | | | | |
|---|---|---|---|---|
| | **Kontrolle** **n = 20** | **Sepsis** **n= 7** | **CAD** **n = 20** | **Schlaganfall** **n = 7** |
| a ($ET_p$ (%)) | | | | |
| *ex vivo* | 1.4±0.5 | 7.7 ± 1.2 * | 7.9 ± 0.6 * | 13.3 ± 3.2 * |
| LPS | 12.1 ± 0.5* | 9.3 ± 0.8 | 9.9 ± 0.6 | 15.2 ± 3.0 |
| B (Plasma-Ceramid (µM) | | | | |
| C24:0 | 2.90 ± 0.62 | 2.1 ± 0.81 * | 2.97 ± 0.83 | 3.0 ± 0.5 |
| C24:1 | 1.58 ± 0.46 | 2.98 ± 0.88 * | 2.31 ± 0.64 * | 2.28 ± 0.35 * |
| C23 | 0.78 ± 0.19 | 0.49 ± 0.08 * | 0.86 ± 0.31 | 1.02 ± 0.3 * |
| C22 | 0.82 ± 0.19 | 1.06 ± 0.23 * | 0.92 ± 0.24 | 0.99 ± 0.23 |
| C16 | 0.69 ± 0.61 | 1.15 ± 0.28 | 0.78 ± 0.25 | 0.72 ± 0.12 |
| **(a)** Die Co-Assemblierung von CD11b und CD14 wurde nach Stimulierung mit LPS (4 nM) und ex *vivo* wie in Beispiel 1 beschrieben analysiert. Die Energietransfer-Werte sind entsprechend der Emission von Cy5 (angeregt bei 488 nm, Akzeptor-sensibilisierte Emission, Gleichung 1) ausgedrückt. Energietransfer-Werte waren bei p < 0,05 (*) signifikant. **(b)** Plasma-Ceramid-Spiegel wurden durch Tandem-Massenspektroskopie quantifiziert und entsprechend als % der Kontrolle ausgedrückt. Unterschiede waren bei p < 0,05 (*) signifikant. | | | | |

**Patentansprüche**

1.  In vitro-Verfahren zur Analyse von exogener und endogener Zell-Aktivierung, umfassend die Messung der Assemblierung von Rezeptoren in einem CD14 umfassenden Rezeptor-Cluster in einer Patientenprobe, wobei die Messung der Assemblierung mittels der Messung eines Energietransfers zwischen den Rezeptoren erfolgt.

2.  Verfahren nach Anspruch 1, wobei der CD14 umfassende Rezeptor-Ctuster außerdem ein oder mehrere LPS-assoziierte Proteine, tntegrin-assoziiene Proteine, G-Protein-Rezeptoren, Komplement-regulierende Proteine, Fcy-Rezeptoren und/oder Scavanger Rezeptoren umfaßt.

3.  Verfahren nach Anspruch 1 oder 2, wobei der Energietransfer zwischen einem Antikörper gegen eines der Proteine (Rezeptoren) nach Anspruch 3 und einem anti-CD14-Antikörper gemessen wird.

4.  Verfahren nach einem der Ansprüche 1 bis 3, wobei der Energietransfer mittels Fluoreszenz-Energietransfer (FRET) gemessen wird.

5.  Verfahren nach einem der Ansprüche 1 bis 4, wobei eine Energietransfer-Effizienz oder ein Energietransfer-Pa-

rameter ($Et_p$) > 5% ein Anzeichen von endogener oder exogener Zell-Aktivierung sind.

6. Verfahren zur Diagnose systemischer Inflammafionen oder einer arteriosklerotischen bzw. entzündlichen Erkrankung der Koronararterien bzw. der Cerebratarterien oder einer Vorstufe dieser Erkrankungen, **dadurch gekennzeichnet, daß** der Energietransfer an Monozyten einer Patientenprobe gemäß dem Verfahren nach einem der Ansprüche 1 bis 5 bestimmt wird, wobei ein Energietransfer ein Anzeichen für eine systemische Koronararterien Inflammation oder eine arteriosklerotische bzw. entzündliche Erkrankung der Cerebralarterien oder Koronararterien oder eine Vorstufe dieser Erkrankungen ist.

**Claims**

1. An *in vitro* method for analyzing exogenous and endogenous cell activation, comprising the measurement of assembling receptors in a CD14-comprising receptor cluster in a patient sample, wherein the assembly being measured by measuring an energy transfer between the receptors.

2. The method according to claim 1, wherein the CD14-comprising receptor cluster also comprises one or several LPS-associated proteins, integrin-associated proteins, G-protein receptors, complement-regulating proteins, Fcy receptors and/or scavenger receptors.

3. The method according to claim 1 or 2, wherein the energy transfer is measured between an antibody against one of the proteins (receptors) according to claim 3 and an anti-CD14 antibody.

4. The method according to any of claims 1 to 3, wherein the energy transfer is measured by means of fluorescence energy transfer (FRET).

5. The method according to any of claims 1 to 4, wherein an energy transfer efficiency or an energy transfer parameter ($Et_p$) > 5 % is an indication of endogenous or exogenous cell activation.

6. The method for diagnosing systemic inflammations or an arteriosclerotic or inflammatory disease of the coronary arteries or cerebral arteries or a preliminary stage of these diseases, **characterized in that** the monocyte energy transfer in a patient sample is determined according to the method of any of claims 1 to 5, an energy transfer being an indication of a systemic inflammation of the coronary arteries or an arteriosclerotic or inflammatory disease of the coronary arteries or the cerebral arteries or a preliminary stage of these diseases.

**Revendications**

1. Méthode d'analyse in vitro d'activation cellulaire exogène et endogène dans un échantillon prélevé sur un patient, comprenant la mesure de l'assemblage de récepteurs dans un groupe de récepteurs comprenant CD14, la mesure de l'assemblage étant effectuée par mesure d'un transfert d'énergie entre les récepteurs.

2. Méthode suivant la revendication 1, dans laquelle le groupe de récepteurs comprenant CD14 comprend en outre une ou plusieurs formes de protéines associées aux LPS, protéines associées à des intégrines, récepteurs des protéines G, protéines de régulation du complément, récepteurs Fcy et/ou récepteurs scavengers.

3. Méthode suivant la revendication 1 ou 2, dans laquelle est mesuré le transfert d'énergie entre un anticorps contre l'une des protéines (récepteurs) selon la revendication 3 et un anticorps anti-CD14.

4. Méthode suivant l'une des revendications 1 à 3, dans laquelle le transfert d'énergie est mesuré au moyen du transfert d'énergie par fluorescence (FRET).

5. Méthode suivant l'une des revendications 1 à 4, dans laquelle un rendement de transfert d'énergie ou un paramètre de transfert d'énergie ($Et_p$) supérieur à 5 % est un indice d'activation cellulaire endogène ou exogène.

6. Méthode pour diagnostiquer des inflammations systémiques ou une pathologie artériosclérotique ou inflammatoire des artères coronaires ou des artères cérébrales ou un stade précurseur de ces pathologies, **caractérisée en ce que** le transfert d'énergie à des monocytes d'un échantillon prélevé sur un patient est déterminée par la méthode selon l'une des revendications 1 à 5, un transfert d'énergie étant un indice d'une inflammation systémique des artères coronaires ou d'une pathologie artériosclérotique ou inflammatoire des artères cérébrales ou des artères coronaires ou d'un stade précurseur de ces pathologies.

Fig. 1 (a)

Fig. 1 (c)

Fig. 1 (b)

Fig. 1 (d)

Fig. 2 (a)

Fig. 2 (b)

Fig. 3 (a)

Fig. 3 (b)

Fig. 4 (a)

Fig. 4 (b)

Fig. 5 a

LPS/LTA induzierte Cluster

ex vivo

Ceramid/HSP70 induzierte Cluster

Fig. 5 b

Toll-like R | FcγIIIa-R (CD16) | FcγI-R (CD64) | FcγII-R (CD32) | CD14 | CD47 | CD55 | β₂ Integrin | CD36

MyD88 | CD81 | PI-4K | PKC | ITAM | G-Protein | UC

HDL | LBP | LPS | SPMase | Cer

TRAF6 | Syk | Src Familie | Pyk2/FAK | Src Familie | 130ᶜᵃˢ | crk / crkl

"Ox. Burst Spreading-Phagozytose"

NIK | Ras | PI-3K | Raf | Rho A

cdc42 / rac | MEK | MLC Ⓟ

IKK | PAK | MAPK | Focale Adkäsions-Phagozytose

JNK | p38

κB/NF-κB

Cytokin Synthese

20